(19) Europäisches Patentamt
European Patent Office
Office européen des brevets

(11) **EP 2 123 774 A1**

(12) **EUROPÄISCHE PATENTANMELDUNG**

(43) Veröffentlichungstag:
**25.11.2009 Patentblatt 2009/48**

(51) Int Cl.:
***C12Q 1/68*** *(2006.01)*

(21) Anmeldenummer: 08009344.6

(22) Anmeldetag: **21.05.2008**

(84) Benannte Vertragsstaaten:
**AT BE BG CH CY CZ DE DK EE ES FI FR GB GR
HR HU IE IS IT LI LT LU LV MC MT NL NO PL PT
RO SE SI SK TR**
Benannte Erstreckungsstaaten:
**AL BA MK RS**

(71) Anmelder: **DR. FALK PHARMA GMBH
D-79108 Freiburg (DE)**

(72) Erfinder:
• **Tewes, Bernhard, Dr.
79108 Freiburg (DE)**
• **Wilhelm, Rudolf, Dr.
76476 Bischweier (DE)**

(74) Vertreter: **Keller, Günter et al
Lederer & Keller
Patentanwälte
Prinzregentenstrasse 16
80538 München (DE)**

(54) **Verfahren zur Charakterisierung der biologischen Aktivität von Helminth-Eiern**

(57)     Beschrieben wird ein Verfahren zur Bestimmung der biologischen Aktivität von Helminth-Eiern, bei der wenigstens eine der nachfolgenden Bestimmungen durchgeführt wird:
a) Bestimmung des Stadiums der Embryonalentwicklung von Helminth-Eiern mit Hilfe der quantitativen PCR-Analyse unter Verwendung geeigneter Marker-Sequenzen zur Ermittlung der Kopienzahl der genomischen DNA,
b) Bestimmung der metabolischen Aktivität von embryonierten Helminth-Eiern,
c) Bestimmung der Induzierbarkeit der Genexpression in embryonierten Helminth-Eiern,
d) mikroskopische Bestimmung der Motilität von im Ei befindlichen Helminth-Larven über lange Beobachtungszeiträume nach Vorinkubation bei erhöhten Temperaturen, und/oder
e) Bestimmung der Schlüpfrate von Helminth-Larven im Versuchstier, wobei die aus dem Darminhalt zurückgewonnenen intakten embryonierten Eier im Vergleich zu einem internen Standard quantifiziert werden.

EP 2 123 774 A1

SEQUENCE LISTING

<110> Dr. Falk Pharma GmbH

<120> Verfahren zur Charakterisierung der biologischen Aktivität von Helminth - Eieren

<130> T.suis

<160> 7

<170> PatentIn version 3.5

<210> 1
<211> 136
<212> DNA
<213> Trichuris suis

<400> 1
tagcagcgac ggcaggtgcc cgtcatcgct ggcaggcagc cggagctgcg gagagcggct      60

aactcagcgc agtacggaag ctgcccgagt tggctacgtc gtcgctacat cgtcgtcagc      120

gtacagcgcg actgag      136


<210> 2
<211> 19
<212> DNA
<213> artificial sequence

<220>
<223> primer

<400> 2
ctgcggagag cggctaact      19


<210> 3
<211> 23
<212> DNA
<213> artificial sequence

<220>
<223> primer

<400> 3
agttggctac gtcgtcgcta cat      23


<210> 4
<211> 16
<212> DNA
<213> artificial sequence

<220>
<223> probe

<400> 4
cagtacggaa gctgcc      16


<210> 5
<211> 63
<212> PRT
<213> Trichuris suis

<400> 5

```
Ile Gly Arg Arg Tyr Asp Asp Ala Ala Val Gln Ser Asp Met Lys His
1               5                   10                  15

Trp Pro Phe Lys Val Val Ser Asp Gly Gly Lys Pro Lys Ile Gln Val
            20                  25                  30

Glu Tyr Lys Gly Glu Thr Lys Met Phe Thr Pro Glu Glu Val Ser Ala
            35                  40                  45

Met Val Leu Val Lys Met Lys Glu Thr Ala Glu Ala Tyr Leu Gly
        50                  55                  60
```

<210>  6
<211>  120
<212>  PRT
<213>  Caenorhabditis elegans

<400>  6

```
Arg Asn Pro Glu Asn Thr Val Phe Asp Ala Lys Arg Leu Ile Gly Arg
1               5                   10                  15

Arg Phe Asp Glu Glu Thr Val Gln Ser Asp Ile Lys His Trp Pro Phe
            20                  25                  30

Thr Val Lys Gly Lys Gln Gly Lys Pro Val Val Glu Val Glu Val Lys
            35                  40                  45

Gly Glu Lys Arg Glu Phe Asn Ala Glu Glu Ile Ser Ala Met Val Leu
        50                  55                  60

Gln Lys Met Lys Glu Thr Ala Glu Ala Val Leu Gly His Ser Val Arg
65                  70                  75                  80

Asp Ala Val Ile Thr Val Pro Ala Tyr Phe Asn Asp Ser Gln Arg Gln
                85                  90                  95

Ala Thr Lys Asp Ala Ala Thr Ile Ala Gly Leu Asn Ala Ile Arg Ile
                100             105                 110

Ile Asn Glu Pro Thr Ala Ala Ala
            115                 120
```

<210>  7
<211>  46
<212>  DNA
<213>  artificial sequence

<220>
<223>  probe

<400>  7
ctccgtcact gaccaccttg aaaggccaat gcttcatgtc agactg                46

**Beschreibung**

Die vorliegende Erfindung betrifft ein Verfahren zur Bestimmung der biologischen Aktivität von Helminth-Eiern in verschiedenen Stadien ihres Lebenszyklus. Das Verfahren ermöglicht, Präparationen von Helminth-Eiern, als wirksame Bestandteile therapeutischer Arzneimittel, kontrolliert herzustellen und eine sichere und therapeutisch effiziente Anwendung am Menschen zu gewährleisten.

Der Einfluss von Parasiteninfektionen auf die Aktivierung des Immunsystems ihrer tierischen Wirte ist bekannt (Review, D M McKay, Parasitology 2006, 132: 1-12). Eine solche Aktivierung beeinflusst auch das Auftreten und den Verlauf von Autoimmunkrankheiten. Epidemiologische Studien zeigen, dass in Regionen mit hohen Raten von Wurminfektionen Autoimmunerkrankungen seltener sind als in Regionen, in denen auf Grund besserer hygienischer Verhältnisse diese Infektionsraten niedriger sind. Cytokinprofile von Patienten mit Morbus Crohn, einer chronisch entzündlichen Darmerkrankung, haben gezeigt, dass Th2-Immunzellen durch Helminthen Infektionen stimuliert werden können. Morbus Crohn, eine Th1-dominierte Autoimmunerkrankung kann durch eine Helminthen Infektion verhindert bzw. beeinflusst werden (Summers et al., Am J Gastroenterol 2003, 98: 2034-2041).

Schon 1971 berichtete Beer (Br. Med. J., 1971, 3: 41), dass *Trichuris suis* ein geeigneter Nematode sein könnte, um eine gezielte, moderate Infektion des Menschen zu erreichen, ohne die pathogenen Effekte hervorzurufen, die beispielsweise mit einer Infektion durch den humanpathogenen Erreger *Trichuris trichiura* einhergehen. Nach neueren Studien scheint die Infektion des Menschen mit *Trichuris suis* nur transient zu sein und die Würmer werden offensichtlich abgestoßen, bevor sie sich vermehren können. Gleichwohl deuten die positiven klinischen Studien zum Einsatz von *Trichuris suis* bei chronisch entzündlichen Darmerkrankungen darauf hin, dass diese transiente Infektion des Menschen eine therapeutisch wirksame Modulation des Immunsystems auslösen kann (Summers et al., Am. J. Gastroenterol. 2003, 98: 2034-2041).

Der Lebenszyklus von *Trichuris suis* beginnt mit dem Absetzen der nicht-embryonierten Eier (L0-Stadium), die vom infizierten Tier mit den Faeces ausgeschieden werden. Im Boden erfolgt über einen Zeitraum von 3-6 Monaten die Embryonierung zum ersten Larven-Stadium L1. In den ersten Tagen nach Erreichen des L1-Stadium lassen sich in den Eiern Larven-Bewegungen verfolgen, danach verfallen die Eier in einen Ruhezustand, in dem sie über Jahre verharren können, ohne jedoch ihre Infektivität zu verlieren. Nach oraler Aufnahme durch einen passenden Wirt schlüpfen die L1-Larven im intestinalen Lumen aus dem Ei und dringen binnen weniger Stunden in die Mucosa des Caecums und Colons ein. In der intestinalen Mucosa durchlaufen die Larven über die nächsten Wochen weitere Stadien der Larval-Entwicklung (L2-L4), bis sie schließlich als adulte L5-Larven wieder in das intestinale Lumen austreten (Beer, Parasitol. 1973, 67: 253-262).

Für die pharmazeutische Herstellung von *Trichuris suis* Eiern (*Trichuris suis ova* = TSO), werden nicht-embryonierte TSO (L0), die entweder *in vitro* oder *in situ* abgelegt wurden, isoliert und mit geeigneten Methoden aufgereinigt (WO 1999 33479, WO 2007 076868, WO 2007 134761). Danach erfolgt die Embryonierung unter kontrollierten Laborbedingungen zu biologisch aktiven (L1) TSO, die den pharmazeutisch aktiven Bestandteil des Arzneimittels darstellen.

Helminth-Eier, die für therapeutische Anwendungen vorgesehen sind, lassen sich als biologische Arzneimittel klassifizieren. Ein zentraler Parameter, der bei Herstellung und Anwendung biologischer Arzneimittel getestet werden muss, ist die biologische Aktivität, welche letztendlich ein Maß für die therapeutische Wirksamkeit des Arzneimittels ist. Ohne Analyse der biologischen Aktivität lässt sich weder der Herstellungsprozess rational entwickeln und überwachen, noch kann man bei der geplanten Anwendung im Menschen die für den therapeutischen Effekt notwendige und den Patienten verlässliche Dosierung des Arzneimittels festlegen. Aus diesem Grunde lassen sich nur solche Helminth-Ei-Präparationen sicher und effektiv als Arzneimittel einsetzen, deren biologische Aktivität hinreichend qualifiziert ist.

Bisher stehen für *Trichuris suis* zwei analytische Verfahren zur Verfügung, die die biologische Aktivität jedoch nur unzureichend charakterisieren:

1. Die Bestimmung der Embryonierungsrate: Die Untersuchung erfolgt mikroskopisch. Anhand morphologischer Kriterien wird beurteilt, ob die Eier eine intakte, vollständig ausgebildete L1-Larve enthalten. Diese Methode erfasst lediglich das Endstadium der Embryonalentwicklung (L1), eignet sich also nicht zur Analyse der Embryonalentwicklung zwischen L0 und L1, die bei der Herstellung durchlaufen wird. Eine weitere Limitierung liegt darin, dass die Embryonierungsrate keine Aussage über die Viabilität und biologische Aktivität der embryonierten L1-TSO bietet. Zudem bedarf die morphologische Beurteilung der Erfahrung des Beobachters und die subjektive Einordnung von morphologischen Grenzfällen limitiert die Richtigkeit und Präzision dieser Methode.

2. Die Bestimmung der Infektionsrate: Für *Trichuris suis* erfolgt die Untersuchung im Schwein. Zu einem bestimmten Zeitpunkt nach Infektion wird die Anzahl der in der Darmschleimhaut etablierten Larven bestimmt und in Relation zur applizierten Dosis an TSO gesetzt (Kringel et al., Vet. Parasitol., 2006, 139: 132-139). Problematisch an der Bestimmung der Infektionsrate ist, dass sie nicht allein von der Funktionalität der Helminth-Eier, sondern gleichermaßen auch von individuellen Wirtsfaktoren, wie beispielsweise dem Immunsystem, der Darmflora und der Darmfunktion der Versuchstiere abhängt. Die Bestimmung der Infektionsrate im Versuchstier ist also intrinsisch mit einer hohen Variabilität verbunden und lässt sich aufgrund des natürlichen Testssystems nicht standardisieren, welches die Eignung als biologischer Aktivitätstest stark einschränkt. Für *Trichuris suis* ist der Test der Infektivität im Schwein mit einer langen Inkubationszeit von mindestens drei Wochen und aufgrund der hohen Variabilität mit einer hohen Tierzahl verbunden, was einen routinemäßigen Einsatz dieses Tests sowohl ethisch wie auch wirtschaftlich fragwürdig erscheinen lässt.

Die WO 2007/134761 beschreibt ein Verfahren zum Nachweis der Viabilität von *Trichuris suis*-Eiern, bei dem der Durchgang der Eier durch die Magen-Darm-Passage eines Schweines in vitro simuliert wird.

Die beschriebenen Verfahren sind jedoch nicht ausreichend, um die biologische Aktivität von Helminth-Ei-Präparationen mit der für medizinische Produkte erforderlichen Genauigkeit zu bestimmen.

Eines der Hauptprobleme eines biologischen Arzneimittels, dessen biologische Aktivität im Tier getestet wird, ist also die Standardisierung des Präparats. Der Einsatz von Tiermodellen, wie die oben erwähnte Infektion von Schweinen, ist äußerst aufwändig und nimmt eine erhebliche Zeit in Anspruch. Im Rahmen der vorliegenden Erfindung wird daher ein Verfahren beschrieben, bei dem verschiedene Arten von Tests durchgeführt werden, die unterschiedliche Aspekte der Entwicklung der Helminth-Eier und ihre biologische Aktivität betreffen. Um zu einer verlässlichen Aussage zu gelangen, muß bei der zu untersuchenden Charge wenigstens einer der erfindungsgemäßen Tests durchgeführt werden, bevorzugt werden jedoch mindestens drei, noch bevorzugter mindestens vier oder sogar fünf der vorliegend beschriebenen Tests durchgeführt, wobei die jeweils relevanten Parameter bestimmt werden. Die Ergebnisse der einzelnen Tests werden insgesamt betrachtet, wobei eine geeignete Helminth-Präparation die vorgegebenen Grenzwerte in jedem Test erfüllen muß, damit hieraus der Schluß gezogen werden kann, dass die Helminth-Ei-Präparation für die pharmazeutische Anwendung geeignet ist.

Es besteht also ein großer Bedarf an einem industriell anwendbaren Verfahren, das die biologische Aktivität von TSO und anderen Helminth-Eiern in verschiedenen Entwicklungsstadien umfassend und zuverlässig analysiert und dabei verschiedene biologische Funktionen der Helminth-Eier charakterisiert. Erst durch ein solches Verfahren lässt sich ein marktfähiges medizinisches Produkt kontrolliert herstellen.

Es ist eine Aufgabe der vorliegenden Erfindung, durch das im Folgenden näher ausgeführte Verfahren die biologische Aktivität von Helminth-Eiern umfassend zu analysieren und so einerseits einen enge Kontrolle des Herstellungsprozesses, andererseits eine sichere und therapeutisch effiziente Anwendung im Patienten zu ermöglichen.

Aus der komplexen Problemstellung wird deutlich, dass eine verlässliche Bestimmung der biologischen Aktivität in der Regel nicht durch einen einzelnen Verfahrensschritt erreicht werden kann. Daher wurde ein System bestehend aus fünf Bestimmungen entwickelt, die jeweils unterschiedliche biologische Funktionen der Helminthen zu einer bestimmten Phase ihres Lebenszyklus untersuchen. Zwar kann es während der einzelnen Herstellungsschritte einer pharmazeutischen Zubereitung ausreichend sein, nur eine Bestimmungsmethode anzuwenden, für die Bestimmung der biologischen Aktivität des Endprodukts müssen jedoch in der Regel wenigstens 3 der nachfolgend in Tabelle 1 dargestellten Bestimmungen durchgeführt werden:

Tabelle 1

| Verfahrensschritt | Beispielhafte Parameter der biologischen Aktivität |
|---|---|
| 1. Bestimmung der Kopienzahl genomischer DNA | • Stadium der Larval-entwicklung zwischen L0 und L1<br>• Identität des Organismus |
| 2. Bestimmung der metabolischen Aktivität | • Viabilität der L1-Larve im Ei |
| 3. Untersuchung der induzierbaren Genexpression | • Aktivierbarkeit der ruhenden L1-Larve |
| 4. Bestimmung des Motilitätsindex | • Bewegungsfähigkeit der L1-Larve im Ei<br>(Voraussetzung für das Schlüpfen) |
| 5. Bestimmung der Schlüpfrate *in vivo* | • Schlüpfen der L1-Larve aus dem Ei<br>(Voraussetzung für die Besiedlung des Wirtes) |

Für eine sichere Charakterisierung der biologischen Aktivität von pharmazeutisch verwendbaren Helminth-Eiern ist es bevorzugt, das komplette Verfahren mit wenigstens drei, bevorzugt 4 und noch bevorzugter allen 5 Schritten durchzuführen. Gegebenenfalls kann es für die Charakterisierung bestimmter Aspekte der biologischen Aktivität oder für die Überwachung von Teilschritten der Herstellung genügen, nur einzelne Schritte des Verfahrens durchzuführen.

Die fünf einzelnen Verfahrensschritte greifen auf Testprinzipien zurück, die in anderem Zusammenhang bereits beschrieben sind. Die Adaption auf embryonierte Helminth-Eier, insbesondere embryonierte Eier von *Trichuris suis,* ist neu und bedurfte der Entwicklung einer Reihe von neuen bisher noch nicht beschriebenen Teilschritten. Auch ist das gesamte Verfahren, das fünf unterschiedliche Aspekte der biologischen Aktivität prüft und so eine umfassende Charakterisierung ermöglicht, neuartig und für Helminth-Eier, insbesondere Eier von *Trichuris suis,* noch nicht zuvor beschrieben worden. Eine weitere Besonderheit dieses Verfahren liegt darin, dass es mit intakten, viablen Helminth-Eiern durchgeführt werden kann und, dass die gemessenen Parameter nur von der Aktivität der Helminth-Eier und nicht von Wirts-Faktoren oder den besonderen Fähigkeiten der den Test durchführenden Person abhängen, somit also objektiver als die bisher angewandten Verfahren sind. Hierdurch ist eine Standardisierung möglich, die für die pharmazeutische Verwendung erforderlich ist.

Durch das Verfahren ist es möglich, die biologische Aktivität von Helminth-Ei-Präparationen mit der für ein pharmazeutisches Produkt erforderlichen Genauigkeit zuverlässig zu bestimmen. Die genaue Bestimmung der biologischen Aktivität von Helminth-Eiern, wie sie in dieser Erfindung beschrieben wird, ist ein wesentlicher Schritt in der Herstellung eines anwendbaren pharmazeutischen Produktes.

Im folgenden werden die 5 Bestimmungsmethoden, die Bestandteil des Verfahrens sind, näher ausgeführt:

**a) Bestimmung der Kopienzahl genomischer DNA**

Bei jeder Zellteilung während der Embryonalentwicklung wird der Chromosomensatz verdoppelt und auf die beiden Tochterzellen verteilt. Daher kann die Kopienzahl der genomischen DNA im Ei als Korrelat für die Anzahl der Körperzellen der sich entwickelnden Larve angesehen werden. Die Bestimmung der Kopienzahl genomischer DNA kann also genutzt werden, um in einem beliebigen Stadium den Status der Embryonalentwicklung der sich entwickelnden Larven abzubilden. Dies ist ein klarer Vorteil gegenüber dem bisher beschriebenen Verfahren (Mikroskopische Bestimmung der Embryonierungsrate, s.o.), mit dem sich nur das Endergebnis der ca. 13 Wochen dauernden Embryonierung untersuchen lässt. Die Embryonierung ist Teil des pharmazeutischen Herstellungsprozesses der Helminth-Eier. Mit der Bestimmung der Kopienzahl genomischer DNA ist erstmals eine prozess-begleitende Analytik möglich. Sie erlaubt zum einen eine rationale Entwicklung des Herstellungsprozesses und die Untersuchung des Einflusses von Prozess-Änderungen und zum anderen die routinemäßige Überwachung der Herstellung während der Embryonierungsphase.

Mit Hilfe der quantitativen PCR-Technologie kann eine Bestimmung der Kopienzahl einer bestimmten Nucleinsäuresequenz in einer Probe durchgeführt werden. Je mehr Kopien in einer bestimmten Nucleinsäuresequenz in der Probe vorhanden sind, umso schneller wird das Amplifikationsplateau erreicht. Durch Eichkurven kann die Anzahl der Kopien relativ genau bestimmt werden. Eine der hier üblicherweise verwendeten Methoden ist die Real Time PCR oder auch die sogenannte TaqMan PCR. Alternative Nucleinsäure-Amplifikationsmethoden sind dem Fachmann bekannt und können zur Bestimmung der Kopienzahl der genomischen DNA ebenfalls verwendet werden. Die genauen Kopienzahlen werden in der Regel mit geeigneten Eichkurven ermittelt.

Als bevorzugten Marker für die genomische DNA von Helminthen eignet sich bevorzugt die ITS1-5.8S-ITS2 Region, die für einen Teil der ribosomalen RNA kodiert. Für *Trichuris suis* (Cutillas et al., Parasitol Res 2001, 100: 383-389) sowie für andere *Trichuris-Arten* und weitere Helminthen ist die Sequenz der ITS1-5.8S-ITS2 Region bereits beschrieben. Eine quantitative PCR (q-PCR), bei der eine Teilsequenz aus der ITS1-5.8S-ITS2 Region amplifiziert wird, eignet sich besonders, um die Kopienzahl der Marker-Region und damit die Kopienzahl der genomischen DNA zu bestimmen. Da die ITS1 und ITS2 Elemente speciesspezifisch sind, ist das Verfahren neben der Untersuchung des Stadiums der Embryonierung auch als qualitativer Identitätsnachweis für den Organismus geeignet.

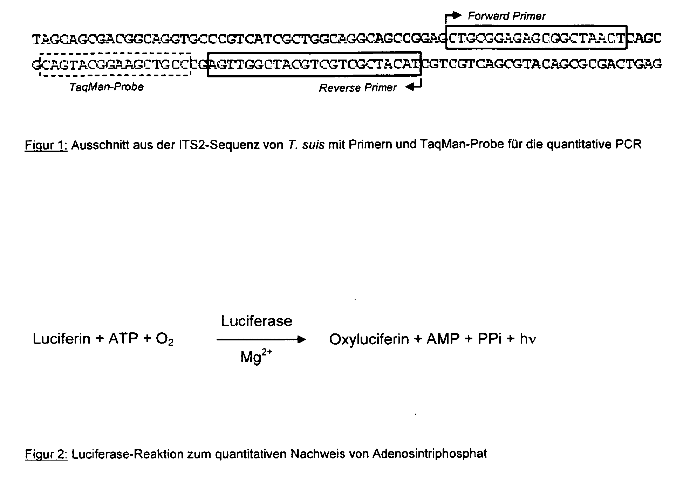

In Figur 1 ist die relevante Gensequenz von *Trichuris suis,* die beispielhaft für die Bestimmung der Kopienzahl genomischer DNA verwendet werden kann, dargestellt. SEQ ID NO:1 zeigt die relevante Gensequenz. Der Vorwärts-Primer ist als SEQ ID NO:2 wiedergegeben, der Rückwärts-Primer als SEQ ID NO:3 und die Sequenz der TaqMan-Probe ist als SEQ ID NO:4 wiedergegeben. Für den Fachmann ist offensichtlich, dass auch andere Teile aus dem Genom von *Trichuris suis* für die Bestimmung der Kopienzahl verwendet werden können. Voraussetzung für die Eignung der Sequenz ist, dass es sich hierbei um eine Nucleotidsequenz handelt, die spezifisch in *Trichuris suis* vorkommt und, dass es keine ähnlichen Sequenzen bei anderen Organismen gibt, die möglicherweise als Kontamination in die zu untersuchende Probe gelangen könnten.

Die Bestimmung der Kopienzahl der genomischen DNA hat auch einen Nebeneffekt. Es kann dadurch bestätigt werden, dass der gewünschte Organismus in der Präparation vorhanden ist und bei Verwendung geeigneter weiterer Sequenzen kann auch der Nachweis geführt werden, ob Kontaminationen mit anderen Organismen vorliegen.

Ein wesentlicher Aspekt, der bei der Bestimmung der Kopienzahl genomischer DNA nicht übersehen werden darf, ist, dass die Helminth-Eier vor der Messung aufgeschlossen werden müssen. Wie in den Beispielen gezeigt hat, sich hierbei ein "Potter-Homogenizer" als besonders geeignet herausgestellt, wobei in bevorzugter Ausführungsform ein Volumen von 2 ml eingesetzt wurde und eine Spaltbreite zwischen 0,01 und 0,03 mm eingestellt wurde. Die Homogenisierung wurder bevorzugt über einen Zeitraum von 5 bis 15 Minuten, bevorzugt etwa 10 Minuten durchgeführt. Der Zellaufschluß wird immer dann durchgeführt, wenn für den jeweiligen Untersuchungsschritt die Zellbestandteile in zugänglicher Form vorliegen müssen.

**b) Bestimmung der metabolischen Aktivität**

Viele Verfahren zur Bestimmung der Viabilität von Zellen basieren auf der Untersuchung der metabolischen Aktivität. Als Energieüberträger und -speicher spielt Adenosintriphosphat eine zentrale Rolle im Zellmetabolismus und kann damit als Marker zur Bestimmung der intrazellulären Stoffwechselaktivität verwendet werden. Die vorliegende Erfindung zeigt, dass der Nachweis dieses Nukleotids auch für die Bestimmung der Viabilität von ausdifferenzierten Organismen wie L1-Larven von *Trichuris suis* verwendet werden kann.

Die Quantifizierung von Adenosintriphosphat in biologischen Systemen wird durch die Messung der Lumineszenz mit Hilfe der Luciferase-Reaktion ermöglicht. Die Lumineszenz ist definitionsgemäß die Emission "kalten" Lichts. Lumineszenz-Systeme basieren auf der chemischen, biochemischen oder elektrochemischen Aktivierung von Substraten, die bei der Rückkehr in ihren Grundzustand einen Teil der Anregungsenergie in Form von Licht abgeben. Beim Nachweis von Adenosintriphosphat werden Luciferasen eingesetzt, die aus Leuchtkäfern (*Photinus pyralis*; *firefly*) isoliert wurden. Das eukaryontische Enzym katalysiert die Oxidation von Luciferin in Anwesenheit von Adenosintriphosphat, Sauerstoff und Magnesiumionen unter Lichtaussendung zu Oxyluciferin. Die Reaktionsgleichung ist in Figur 2 dargestellt.

Voraussetzungen für eine erfolgreiche Etablierung der Adenosintriphosphat-Bestimmung in embryonierten viablen Eiern von *Trichuris suis* sind die Stimulierung einer für die Lumineszenzmessung ausreichenden Adenosintriphosphat-Synthese in den L1-Larven, ein vollständiger Aufschluss der Eier durch ein geeignetes Homogenisierungsverfahren zur Freisetzung des intrazellulär gebildeten Nukleotids sowie die quantitative und damit störungsfreie Erfassung von Adenosintriphosphat aus der komplexen Matrix. Weiterhin ist für die Differenzierung von lebenden und toten L1-Larven ein effektives Inaktivierungsverfahren erforderlich, so dass der Basiswert von Adenosintriphosphat in abgetöteten Organismen reproduzierbar bestimmt werden kann.

Die Bestimmung des ATP-Gehaltes zur Untersuchung der metabolischen Aktivität von Helminth-Ei-Populationen ist erfindungsgemäß bevorzugt.

Zur Untersuchung der metabolischen Aktivität einzelner Helminth-Eier, sind auch Färbemethoden mit Tetrazoliumverbindungen geeignet, die ursprünglich für frei zugängliche tierische Zellen in Zellkulturen oder histologische Schnitte entwickelt wurden. Die Tetrazoliumverbindungen werden in metabolisch aktiven Zellen durch die Wirkung mitochondrialer Enzyme zu farbigen Formazanen reduziert, die sich in den Zellen ablagern. Entscheidend für die Übertragung der Färbemethoden von frei zugänglichen tierischen Zellen auf die in Eiern befindlichen vielzelligen L1-Larven von Helminthen ist eine Vorbehandlung der Eier, die eine Permeation des Substrates erlaubt ohne die Viabilität der sich im Ei befindenden Larve zu beeinträchtigen. Die L1-Larven von *Trichuris suis* sind von einer rigiden Schale umgeben, die den Stoffaustausch mit der Umgebung stark einschränkt und die Permeation der für die Untersuchungen notwendigen Substanzen verhindert.

Als vorteilhaft für den schonenden Abbau der Ei-Hülle hat sich die Behandlung der Eier mit hypochloriger Säure erwiesen, die schon zuvor beschrieben worden ist (Beer, Parasitol., 1973, 67: 263-278) und die gegebenenfalls durch einen nachfolgenden enzymatischen Verdau mit Chitinase und Protease unterstützt werden kann.

Um die metabolische Aktivität bestimmen zu können, muß ein "Nullwert" ermittelt werden. Hierbei handelt es sich um einen Vergleichswert, von dem aus die jeweilige metabolische Aktivität ermittelt wird. In bevorzugter Ausführungsform wird der "Nullwert" mit inaktiven Helminth-Eiern ermittelt.

Als geeignetes Verfahren zur Inaktivierung der Eier hat sich die Kryoinaktivierung (Schockgefrieren und Lagerung bei -80°C über 24 Stunden) herausgestellt. Das Adenosintriphosphat-Signal von auf diese Weise inaktivierten Proben konnte nahezu auf Hintergrundrauschen reduziert werden. Die Herstellung kryoinaktivierter Ei-Proben erfolgt in Phosphat-gepufferter physiologischer Kochsalzlösung. Die Kryoinaktivierung kann selbstverständlich auch bei den anderen Bestimmungsverfahren eingesetzt werden.

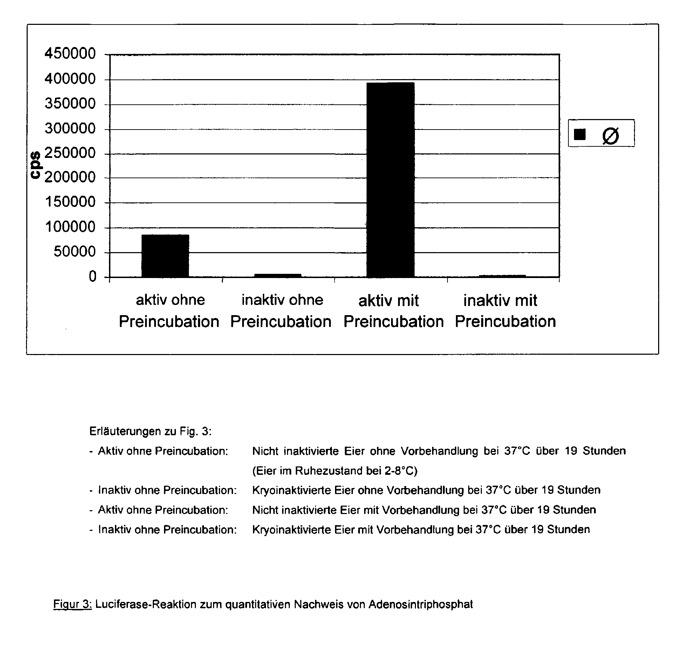

Figur 3 zeigt beispielhaft die Wirkung der Kryoinaktivierung beim quantitativen Nachweis von Adenosintriphosphat mit Hilfe der Luciferase-Reaktion. Durch die Kryoinaktivierung kann spezifisch die Wirkung der Präinkubation gemessen werden. Bei inaktivierten (abgetöteten) Helminth-Eiern ist auch nach Präinkubation kein Anstieg von Adenosintriphosphat erkennbar. Biologisch aktive Helminth-Eier weisen dagegen eine Aktivität auf, die durch den Anstieg des Adenosintriphosphats nach einer Vorbehandlung bei 37°C über eine Zeitdauer von 19 Stunden erzielt wird.

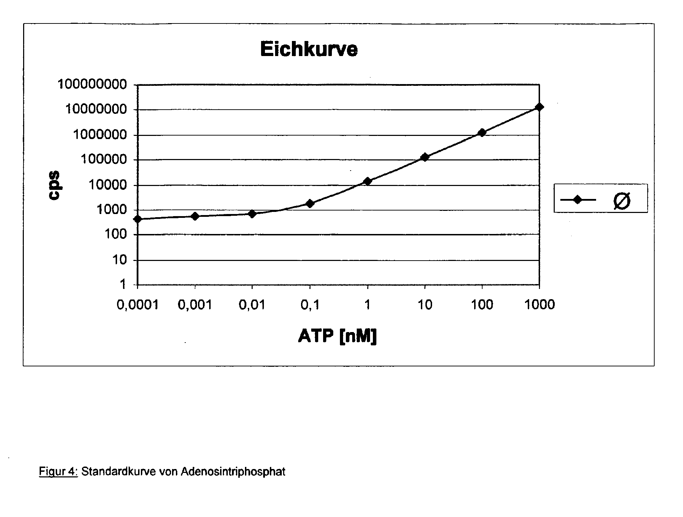

In einer bevorzugten Ausführungsform werden für jeden Ansatz 5500 Eier in 550 µl Lösung verwendet. Nach der Vorinkubation werden die Eier durch Zentrifugation abgetrennt (5 min bei 500 UpM) und in 225 µl Lysepuffer resuspendiert. Zur Freisetzung des Zellinhalts mit dem durch Inkubation gebildeten Adenosintriphosphats werden die Eier mit einem Potter-Homogenizer (Volumen 2 ml; Spaltbreite 0.01-0.03 mm) für 10 min homogenisiert. Nur dieses Verfahren führt zu einem vollständigen Aufschluss der Eier und damit zu einer quantitativen Freisetzung des Nukleotids. Um Adenosintriphosphat-spaltende Hydrolasen zu inaktivieren, erfolgt der Aufschluss der Eier mit einem Lysepuffer (Bestandteil: Phosphorsäure pH 2). Von den einzelnen Ansätzen werden jeweils 50 µl auf 96-Mikrowellplatten auspipettiert und jeder Ansatz mit weiteren 100 µl Phosphatpuffer verdünnt. Durch Zugabe von 100 µl des kommerziell erhältlichen Adenosintriphosphat-Kits wird die Luciferase-Reaktion gestartet. Nach einer Inkubation von 2 Minuten wird die Mikrowellplatte im Luminometer ausgelesen und die Lumineszenz bestimmt (Fig. 3). Die Quantifizierung erfolgt gegen eine Standardkurve von Adenosintriphosphat in Phosphatpuffer (1.0 µM, 0.1 µM, 10 nM, 1 nM, 0,1 nM, 10 pM, 1,0 pM, 0,1 pM). Eine typische ATP-Eichkurve ist in Figur 4 dargestellt.

Durch gezieltes Aufstocken von Adenosintriphosphat zu homogenisierten kryoninaktivierten Proben von embryonierten Eiern konnte innerhalb des linearen Bereichs der Standardkurve (1 bis 1000 nM) eine Wiederfindung von 84% erzielt werden. Eine relevante Wechselwirkung der komplexen Matrix des Ei-Homogenats mit der Luciferasereaktion und der Lumineszenz ist damit nicht zu erkennen.

Das Beispiel zeigt eindeutig, dass die Lumineszenzmessung eingesetzt werden kann, um viable embryonierte Eier von *Trichuris suis* von inaktivierten und damit nicht viablen Eiern zu differenzieren. Die erfolgreiche Durchführung des Tests gelingt jedoch nur mit einer Kombination von optimierten Verfahrensparametern bestehend aus Proben-Aktivierung, Proben-Homogenisierung und der Herstellung geeigneter Kontrollen durch ein optimiertes Inaktivierungsverfahren. Der geeignete ATP-Gehalt biologisch aktiver Helminth-Eier liegt in einem Bereich von mindestens 0,01 pmol ATP pro Ei.

### c) Untersuchung der induzierbaren Genexpression

Das Prinzip dieses Verfahrens liegt in der Induktion der Gen-Expression, die nur in lebenden Zellen stattfinden kann und mit deren Hilfe zwischen lebenden und toten Larven differenziert werden kann. Zudem ist die Induzierbarkeit der Genexpression vermutlich Voraussetzung dafür, dass die ruhende L1-Larve verschiedene Aktivierungszustände durchlaufen kann, die notwendig sind, die nächsten Stadien des Lebenszyklus (Schlüpfen, Etablierung in der Mucosa etc.) zu induzieren.

Als besonders vorteilhaft erscheint die Untersuchung der Expression von Hitzeschock-Proteinen, da sich diese in einfacher Weise durch Temperatur-Erhöhung induzieren lassen und die gebildete Messenger-RNA (mRNA) im allgemeinen stabil gegen einen schnellen Abbau ist. Im Rahmen der vorliegenden Erfindung gelang in *Trichuris suis* erstmals der Nachweis einer Gen-Sequenz, die weitgehend homolog zum Hitzeschock-Protein hsp70 aus dem Helminthen *Caenorabhditis elegans* ist.

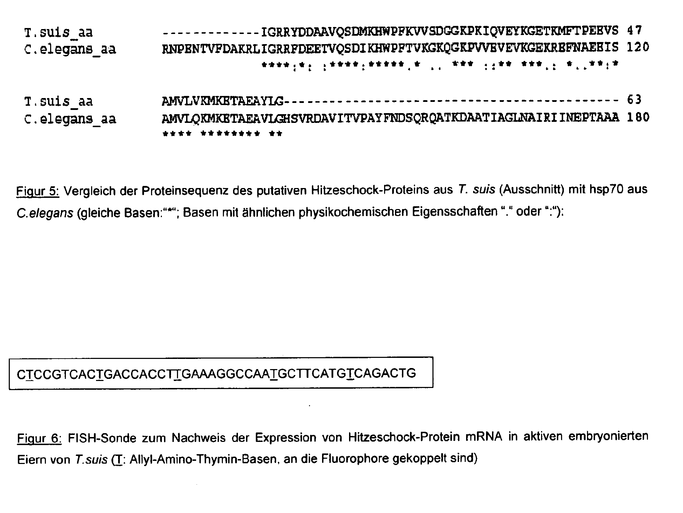

In einer bevorzugten Ausführungsform der vorliegenden Erfindung wird die Genexpression des Hitzeschockproteins von *T.suis* analysiert. In Figur 5 ist die Proteinsequenz von *Trichuris suis* (SEQ ID NO:5), dargestellt im Vergleich mit der Aminosäuresequenz des homologen Proteins aus *Caenorabhditis elegans* (SEQ ID NO:6).

Die induzierbare Genexpression kann durch Bestimmung des Gehalts an für ein bestimmtes induzierbares Protein kodierende Messenger-RNA erfolgen. Aus der Aminosäuresequenz kann die Nucleotidsequenz der Messenger-RNA abgeleitet werden. Für den Fachmann stellt es kein Problem dar, geeignete Vorwärts- und Rückwärts-Primer festzulegen und dazwischen die Sequenz einer TaqMan-Probe festzusetzen. Mit Hilfe einer Real Time PCR kann dann die Induktion des Gens bestimmt werden. Voraussetzung für eine aussagekräftige Bestimmung ist, dass auch eine Kontrollprobe gemessen wird, die abgetötete Wurmeier enthält. Außerdem muss es sich um ein Protein handeln, das einfach und verlässlich induzierbar ist. Eines der Beispiele ist das Hitzeschockprotein, es können jedoch ebenso gut auch andere durch bestimmte Reize induzierbare Gene für die Bestimmung herangezogen werden.

Der Nachweis der exprimierten Messenger-RNA auf Ebene einzelner Eier gelingt auch durch Fluoreszenz-in situ-Hybridisierung (FISH) mit einer geeigneten Gen-Sonde. Mit besonderem Vorteil lässt sich die Ei-Population anschließend mithilfe Durchflußcytometrie schnell und zuverlässig untersuchen.

Die Sequenz einer geeigneten Sonde zur in situ-Hybridisierung ist in Figur 6 (SEQ ID NO:7) dargestellt.

### d) Bestimmung der Motilität von Larven in intakten Helminth-Eiern

Das Prinzip des Verfahrens beruht auf dem Nachweis von Körperbewegungen als Parameter für die Funktionalität der L1-Larve im Ei. Die Motilität der Larve ist Voraussetzung dafür, dass sie unter passenden Umgebungsbedingungen schlüpfen kann. Bei frei lebenden Larven und Würmern wie *C.elegans* gilt die Motilitäts-Untersuchung als zuverlässiger Viabilitäts- und Funktionsnachweis.

Im Rahmen der vorliegenden Erfindung wurde überraschend und erstmals am Beispiel von *Trichuris suis* gefunden, dass sich auch bei nicht frei lebenden, im Ei befindlichen Helminth-Larven durch exakte Einstellung der Umgebungstemperatur subtile Bewegungen induzieren lassen. Die Bewegungen der Larven im Ei sind sehr langsam und nur detektierbar, wenn die Eier wie in der vorliegenden Erfindung offenbart im Zeitraffer über einen langen Zeitraum mikroskopiert werden. In Kombination mit einer Bildanalyse-Software lässt sich der mikroskopische Motilitätstest automatisieren. Der Motilitätsindex als Parameter für die biologische Aktivität der Eier berechnet sich wie folgt:

$$Motilit\ddot{a}ts - Index = \frac{Anzahl\ der\ motilen\ Larven\ im\ Beobachtungsfeld}{Anzahl\ der\ untersuchten\ Eier\ im\ Beobachtungsfeld}$$

Bei der Bestimmung der Motilität von Larven ist eine exakte Temperierung für den Erfolg der Methode entscheidend. Zunächst werden die Eier über einen Zeitraum von 2 bis 30 Stunden, bevorzugt 4 bis 20 Stunden bei einer exakt eingestellten Temperatur, die zwischen 36°C und 42°C, bevorzugt 37°C bis 41°C und besonders bevorzugt bei 39,5°C liegt, vorinkubiert. Nach dieser Vorinkubation werden die Larven in ein geeignetes Mikroskop verbracht, wobei die eingestellte Temperatur zumindest annähernd beibehalten werden kann. Die Beobachtung erfolgt dann mit einer Zeitrafferaufnahme über einen Zeitraum von 2 Minuten bis 4 Stunden, bevorzugt 30 Minuten bis 2 Stunden bei einer Temperatur zwischen 36°C und 42°C, bevorzugt 38°C bis 40°C.

Figur 7 zeigt die Ergebnisse eines erfindungsgemäß bestimmten Motilitätsindex, wobei bei inaktivierten Wurmeiern keinerlei Motilität beobachtet werden kann, bei den aktiven Wurmeiern steigt dagegen der Motilitätsindex mit der Zeit an.

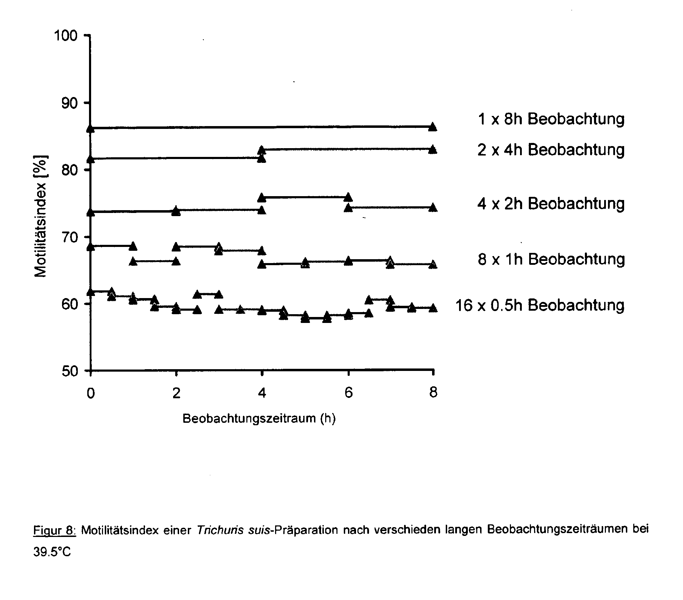

Figur 8 zeigt den Motilitätsindex im Zusammenhang mit verschiedenen Beobachtungszeiträumen.

**e) Bestimmung der Schlüpfrate im Intestinum**

Das Prinzip dieses Verfahrens-Schritts besteht in der Bestimmung der Schlüpfrate von Helminth-Larven im Intestinum von Versuchtieren oder alternativ im Darminhalt, der zuvor aus Versuchstieren entnommen wurde.

Die Schlüpfrate kann in einer Ausführungsform in Darminhalt bestimmt werden, der Versuchstieren entnommen wird, und zwar bevorzugt am Beginn des Colons bzw. Ende des Duodenums. Die Schlüpfrate kann dann in dem Darminhalt bestimmt werden, ohne dass die Schlüpfrate direkt in einem Versuchstier bestimmt werden muss.

In dem neu entwickelten Verfahrensschritt wird in relativ kurzem Abstand nach Inokulierung der Darminhalt analysiert und die intakten Eier sowie die nach dem Schlüpfen zurückgelassenen Ei-Hüllen gezählt. Wesentlicher Vorteil gegenüber der im Stand der Technik beschriebenen Bestimmung der Infektivität ist, dass mit dem Schlüpfen nur die erste Phase des Lebenszyklus untersucht wird, die von individuellen Wirtsfaktoren nur wenig beeinflusst wird.

Überraschend konnte für *Trichuris suis* erstmals gezeigt werden, dass die Larven nicht nur im passenden Wirt, dem Schwein, sondern auch im Kaninchen quantitativ schlüpfen. Damit steht beispielsweise für *Trichuris suis* ein Versuchstier mit geringeren intestinalen Dimensionen zur Verfügung, was die Wiederfindung der mikroskopisch kleinen Eier und Ei-Hüllen deutlich erleichtert.

Überraschend wurde gefunden, dass sich mit kommerziellen für die Markierung von Proteinen entwickelten Fluoreszenzfarbstoffen die Ei-Schale dauerhaft anfärben lässt. Die Kopplung von Fluoreszenzfarbstoffen an die Ei-Hülle erleichtert wesentlich die Wiederfindung der Eier, da der Darminhalt fluoreszenzmikroskopisch untersucht werden kann.

Eine weitere wesentliche Neuerung ist der Einsatz von nichtembryonierten oder inaktivierten Helminth-Eiern als interner, unveränderlicher Standard. Diese haben die gleiche Transit- oder Verweilzeit im Darm, bleiben jedoch im Unterschied zu den embryonierten und biologisch aktiven Eiern bei der Darmpassage intakt. Durch Markierung der als interner Standard vorgesehenen Eiern mit einem zweiten (unterschiedlichen) Fluoreszenzfarbstoff kann leicht zwischen dem internen Standard und den zu untersuchenden Eiern unterschieden werden.

Zur Analyse werden im Darminhalt zu einem geeigneten Zeitpunkt nach Inokulierung die Anzahl der mit Fluoreszenzfarbstoff 1 markierten intakten Eier und leeren Ei-Hüllen der zu untersuchenden Probe sowie die mit Fluoreszenzfarbstoff 2 markierten intakten Eier des internen Standards untersucht. Die Schlüpfrate im Intestinum lässt sich wie folgt auf Basis der intakten (nicht geschlüpften) Eier berechnen.

Formel zur Berechnung der Schlüpf-Rate:

$$Schl\ddot{u}pf - Rate = 1 - \frac{\frac{[IE]i}{[IS]i}}{\frac{[IE]0}{[IS]0}}$$

mit:
- [IE]=Anzahl der intakten Eier aus der zu untersuchenden Probe (mit Farbstoff 1 markiert)
- [IS]=Anzahl der intakten Eier des internen Standards (mit Farbstoff 2 markiert)
- i = intestinale Probe
- 0 = Inokulierungsprobe

In der nachfolgenden Tabelle 2 sind die bevorzugten Bestimmungen und die damit bevorzugt erzielbaren Werte zusammengefasst, die bevorzugte Grenzwerte darstellen, ob die untersuchte Präparation die erforderliche biologische Aktivität aufweist.

Tabelle 2

| bevorzugte Bestimmung | Parameter | Zielgröße |
|---|---|---|
| a) PCR, genomische DNA | Kopienzahl genomischer DNA/Ei | Bereich: 50-1200 Kopien/Ei an d28 (Zu Beginn der Embryonierung liegt die Kopienzahl bei 1 Kopie/Ei; nach 28 Tagen Embryonierung sollte mindestens eine Kopienzahl von 50/Ei erreicht sein.) |
| b1) Metabolische Aktivität Variante 1: ATP Gehalt | Mittlerer ATP-Gehalt/Ei | Bereich: > 1 pmol ATP/embroyoniertes Ei |
| b2) Metabolische Aktivität Variante 2: Aktivitätsfärbung | Anteil der Eier mit positiver Aktivitätsfärbung | Bereich: ≥ 50 % (min. 50 % der Eier sollten eine positive metabolische Aktivitätsfärbung aufweisen) |
| c) Induzierbare Genexpression | Anteil der Eier, die eine induzierbare Genexpression zeigen | Bereich: ≥ 50 % (bei min. 50 % der Eier sollte sich die Genexpression induzieren lassen) |
| d) Motilitätsindex | Anteil der Eier, die eine motile Larve enthalten | Bereich: ≥ 50 % (min. 50 % der Eier sollten eine motile Larve enthalten) |
| e) Schlüpfrate | Anteil der Eier, die im Intestinum schlüpfen | Bereich ≥ 40 % (aus min. 40 % der verabreichten embryonierten Eier sollte eine Larve schlüpfen) |

**Beispiele**

Beispiel 1:

Auf Basis der ITS2 Sequenz von *Trichuris suis* wurde mit Hilfe des TaqMan-Systems eine quantitative PCR-Methode entwickelt. Die Zielsequenz, die Primer und die TaqMan-Probe sind nachfolgend dargestellt. Die Zielsequenz wurde so gewählt, dass sie eine Differenzierung zwischen *Trichuris suis* und anderen *Trichuris*-Arten ermöglicht, deren Sequenz ebenfalls bekannt ist. So liefert der Test neben der Information über die ITS2-Kopienzahl auch den qualitativen Nachweis, dass es sich bei dem untersuchten Organismus tatsächlich um *Trichuris suis* handelt.

Zur Probenvorbereitung werden die Eier (1000 Eier in 500 μl Lösung) mit einem Potter-Homogenizer (Volumen 2ml; Spaltbreite 0.01-0.03 mm) für 10 min homogenisiert. Die mikroskopische Kontrolle belegt, dass sich überraschenderweise mit dieser Methode die Ei-Hüllen vollständig aufbrechen lassen, während andere bei der Genisolierung übliche Aufschlußverfahren bei Eiern von *Trichuris suis* erfolglos bleiben. Nach Zusatz von 1.54 μg Fisch-Sperma-DNA wird das Ei-Homogenat mit einem kommerziellen Kit zur Genisolisierung (DNeasy-Blood and Tissue-Kit; Qiagen) nach Vorschrift des Herstellers umgesetzt. Mit dem DNA-Isolat (Gesamtvolumen 50 μl) wird nach dem in der folgenden Tabelle dargestellten Protokoll eine PCR-Reaktion durchgeführt.

Tab. 3: Protokoll der PCR-Reaktion für den Zielabschnitt aus der ITS2-Region aus *T. suis*

| | |
|---|---|
| Instrument: | AB7900HT (Applied Biosystems) |
| Enzyme: | TaqMan Gene Expression Master Mix (Applied Biosystems) |
| Primer, TaqMan-Probe: | s. Figur 1 |
| Finale Konzentration Primer: | 900 nM |
| Finale Konzentration TaqMan Probe: | 200 nM |
| Proben-Volumen: | 5 μl |
| Gesamtvolumen PCR-Reaktion: | 25 μl |

(fortgesetzt)

| Instrument: | AB7900HT (Applied Biosystems) |
|---|---|
| Temperatur-Programm: | 50°C / 2 min<br>95°C / 10 min<br>40 Zyklen [95°C / 9 sec; 60°C / 1 min] |

Die Amplifikations-Effizienz der Methode liegt zwischen 92% und 98% und die Sensitivität ist groß genug, um die Kopienzahl der ITS2-Gene in einem einzelnen Ei bestimmen zu können.

Mit der PCR-Methode wurde die ITS2-Kopienzahl von nicht-embryonierten und vollständig embryonierten *Trichuris suis* Eiern untersucht. Die Ergebnisse sind in der folgenden Tabelle dargestellt.

Tab. 4: Quantitative PCR für die ITS2-Region in nicht-embryonierten und embryonierten Eiern von *Trichuris suis*

| Probe (jeweils 1000 Eier) | Ct-Wert | Rel. Kopienzahl ITS2 |
|---|---|---|
| Nicht-embryonierte TSO (L0) | 25,5 | 1 |
| Embryonierte TSO (L1) | 15,5 | 1024 |

(Ct-Wert (Cycle treshold): Anzahl der PCR-Zyklen, die nötig sind, um ein Fluoreszenzsignal zu erreichen, das sich signifikant von der Background-Fluoreszenz unterscheidet)

Die PCR-Analyse zeigt, dass die ITS2-Kopienzahl in embryonierten TSO etwa um den Faktor 1000 höher liegt als die ITS2-Kopienzahl in nicht-embryonierten TSO. Da das nicht-embryonierte Ei (L0) als einzelne Zelle betrachtet werden kann, bedeutet dies, dass die L1-Larve von *T.suis* ca. 1000 somatische Zellen besitzt. Für *T.suis* ist die Anzahl der somatischen Zellen in der L1-Larve bisher nicht bekannt; Für den verwandten Organismus *C.elegans* wurde wurde eine Größe von 959 somatischen Zellen gefunden, was gut mit den hier gefundenen ca. 1000 Zellen übereinstimmt.

Um zu prüfen, ob sich mit dem Testverfahren verschiedene Stadien der Larvalentwicklung untersuchen lassen, wurden TSO über 4 Wochen embryoniert und regelmäßig mit der ITS2-PCR untersucht (Tab. 5).

Tab. 5: Relative ITS-2 Kopienzahl in Abhängigkeit von der Dauer der Embryonierung

| Embryonierungsdauer | Ct (T) - Ct (0) | Rel. Kopienzahl ITS2 |
|---|---|---|
| 0 Tage (L0-Zustand) | 0 | 1 |
| 7 Tage | 2,52 | 6 |
| 14 Tage | 6,04 | 66 |
| 21 Tage | 7,37 | 165 |
| 28 Tage | 7,76 | 216 |
| 90 Tage (L1-Zustand) | 10,0 | 1024 |

Das Beispiel zeigt eindeutig, dass das Verfahren genutzt werden kann, um verschiedene Stadien der Larval-Entwicklung zwischen L0 und L1 anhand der Anzahl der somatischen Zellen zu charakterisieren. Somit eignet sich dieses Verfahren besonders um den Verlauf der Embryonierung prozess-begleitend zu analysieren. Als besonders bevorzugte Spezifikation bei der Herstellung einer Helminth-Ei-Präparation erscheint das Erreichen einer relativen Kopienzahl von 50 - 1200 innerhalb der ersten 28 Tage der Embryonierung.

Beispiel 2:

Für den Nachweis von Adenosintriphosphat stehen kommerziell erhältliche Kits zur Verfügung, die unter anderem die für die Reaktion erforderlichen Luciferase-Enzyme sowie das Substrat Luciferin und Magnesiumsalze enthalten. Die Stimulation der Adenosintriphosphat-Bildung durch Vorinkubation der embryonierten Eier ist zwingend erforderlich. Der Adenosintriphosphat-Gehalt von viablen embryonierten Eier im Ruhezustand erlaubt keine ausreichende Differenzierung zu toten Eier (Figur 3). Dabei hat sich ein Verfahren als geeignet erwiesen, bei dem die Eier über einen Zeitraum von 19 Stunden bei 37°C inkubiert werden. Ein wesentlicher Vorteil des entwickelten Analysenverfahrens besteht darin, dass für diese Inkubation keine Probenvorbereitung erforderlich ist. Die vorliegenden Ei-Suspensionen können unabhängig von der qualitativen und quantitativen Zusammensetzung des Mediums direkt verwendet werden. Selbst stark saure Medien mit pH-Werten <2 können ohne Probenvorbereitung eingesetzt werden und haben auf die Stimulierung von Adenosintriphosphat in den Eiern keine negative Auswirkung. Durch die Inkubation wird eine eindeutige Unterscheidung zwischen lebenden und toten F1-Larven ermöglicht (Fig. 3).

Beispiel 3:

Mit Hilfe von Primern, die aus der Sequenz des in *Caenorhabditis elegans* gefundenen Hitzeschock-Proteins hsp70 und homologen Sequenzen aus *Trichuris muris* und *Trichuris vulpis* abgeleitet wurden, ließ sich in einem RNA-Isolat aus *Trichuris suis* eine Gensequenz amplifizieren. Die neue Sequenz aus *Trichuris suis* weist, übersetzt in die Protein-Sequenz, eine 65% Übereinstimmung mit der Proteinsequenz des hsp70-Proteins aus C.elegans auf (Fig. 5).

Für das putative Hitzeschock-Protein aus *T.suis* wurde eine quantitative RT-PCR-Methode entwickelt. Die Quantifizierung erfolgte im Vergleich zur konstitutiv exprimierten 18S-rRNA. Aktive embryonierte Eier von *T.suis* wurden einem Hitzeschock (20 min bei 45°C) unterzogen und die Expression des Hitzeschock-Proteins wurde mit der in unbehandelten aktiven *T.suis* verglichen.

Tab. 6: Relative Expression des Hitzeschock-Proteins in aktiven embryonierten *T.suis*-Eiern (L1-Larven) (2 unabhängige Experimente mit jeweils 3 Proben; Fehler: Standard-Abweichung):

|  | Experiment #1 | Experiment #2 |
|---|---|---|
| Kontrolle | 1.075 $\pm$ 0.072 | 1.040 $\pm$ 0.029 |
| *T.suis* 45°C, 20min | 1.690 $\pm$ 0.212 | 1.467 $\pm$ 0.049 |

Die relative Expressionsanalyse zeigt eindeutig, dass sich die Expression der hsp-70 ähnlichen mRNA in *T.suis* durch Hitzeschock induzieren lässt (Tab. 6). Somit steht für den Aktivitätstest ein Gen zur Verfügung, dessen Expression sich durch Wahl geeigneter Versuchsbedingungen aktiv induzieren lässt.

Der Nachweis der aktiven Genexpression am einzelnen Ei gelingt mit Hilfe der Technik der Fluoreszenz-*in situ*-Hybridisierung (FISH). Um das putative Hitzeschock-Protein nachweisen zu können, wurde beipielsweise die in Figur 6 dargestellte Fluoreszenzmarkierte DNA-Sonde entwickelt (SEQ ID NO:7).

Nach Fluoreszenzmarkierung der aktiven Eier kann die Analyse der Ei-Population auch mittels der Durchfluß-Cytometrie untersucht werden. Gegenüber der mikroskopischen Analyse besteht der Vorteil darin, dass die Analyse schneller und objektiver erfolgt.

Das Beispiel zeigt eindeutig, dass sich mit dem hier beschriebenen Verfahren die Induktion der Genexpression in (einzelnen) Helminth-Eiern untersuchen lässt. Damit steht erstmals ein Verfahren zur Verfügung, mit dem sich die Aktivierbarkeit ruhender L1-Larven von Helminthen untersuchen lässt.

Wird die Induktion der Genexpression als biologischer Aktivitätsnachweis bei der Herstellung von Helminth-Ei-Präparationen genutzt, so ist ein geeignetes Akzeptanzkriterium bei 25%-100% positiver Eier, bevorzugt 50 - 100 % positive Helminth-Eier.

Beispiel 4:

4.1 Abhängigkeit des Motilitäts-Index von den Untersuchungsbedingungen

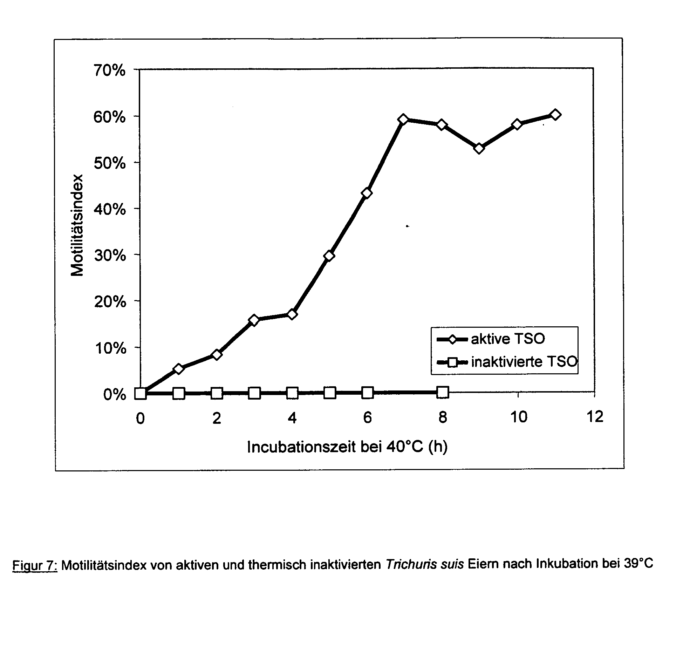

Aktive sowie durch Inkubation bei 48°C über 72 Stunden inaktivierte *Trichuris suis*-Eier wurden über 11 Stunden bei 39°C inkubiert. Stündlich wurden die Eier für jeweils 5 min beobachtet und der Motilitäts-Index bestimmt. Die Figur 7 zeigt die Entwicklung des Motilitäts-Index mit der Zeit.

Das Beispiel verdeutlicht, dass eine relative lange Vorinkubation bei erhöhten Temperaturen (hier: 7 Stunden bei 39°C) notwendig ist, um die Larven aus dem Ruhezustand zu aktivieren und einen konstant hohen Motilitäts-Index zu messen. Diese Beobachtung war unerwartet, da die fertig embryonierten im Ei befindlichen L1-Larven bisher als immobil galten (Parasitology 1973, 67: 253-262). Wahrscheinlich wurde die Motilität der Larven im Ei bisher nicht beobachtet, weil eine lange Vorinkubation notwendig ist und die langsamen Bewegungen nur im Zeitraffer sichtbar sind.

Der Vergleich von aktiven und inaktivierten Eiern zeigt eindeutig, dass sich durch Untersuchung der Motilität in einfacher Weise zwischen biologisch aktiven und inaktiven *Trichuris suis* Eiern differenzieren lässt.

Neben der Aktivierungsphase beeinflusst die Länge des Beobachtungsfensters den gemessenen Motilitätsindex, da die Larven sich nicht synchron bewegen und somit ein längeres Beobachtungsfenster die Wahrscheinlichkeit erhöht; in einem vitalen Ei die sporadischen Bewegungen zu erfassen. Dies zeigt die Untersuchung (Figur 7), bei der eine *Trichuris suis* Präparation nach Aktivierungsphase (8 Stunden bei 37°C) über verschieden lange Zeiträume bei 39.5°C bis 40°C beobachtet wurde. Die Untersuchung zeigt auch, dass die Motilität über die gesamte Beobachtungsperiode von 8 Stunden stabil bleibt.

Charakteristisches und neuartiges Merkmal dieses Verfahrens sind also die Aktivierungsphase mit exakter Temperaturkontrolle sowie das lange Beobachtungsfenster. Das Beispiel 4 zeigt, dass sich in ruhenden L1-Larven, die sich noch im Ei befinden, durch geeignete Temperatur- und Beoachtungs-Bedingungen Bewegungen induzieren und messen lassen. Mit dem Verfahren lässt sich auf einfache Weise die Motiliät als Vorrausetzung für das Schlüpfen und damit als Parameter für die biologische Aktivität der Eier bestimmen und eindeutig zwischen aktiven und inaktiven L1-Larven differenzieren.

4.2 Vorteilhafte Testbedingungen

Als vorteilhaft haben sich die folgenden Test-Bedingungen erwiesen: Eine Probe mit 15.000 Helminth-Eiern in 300 $\mu$l Phosphatpuffer, pH 7.4 wird in die Kammer einer 96-well-Platte überführt (Bodenfläche: 0,31 cm$^2$). Bei dieser Einsaatdichte befinden bei 200-facher Vergrößerung ca. 80-150 Eier im Sichtfeld des Mikroskops. Nach einer 8-stündigen Inkubation bei 37°C (Aktivierungsphase) wird die Temperatur auf 39,5°C erhöht und es werden nacheinander 4 Beobachtungsfelder ausgewählt und für jeweils 2 Stunden beobachtet. Dabei wird ein Film mit 3 Bildern pro Minute aufgezeichnet. Anschließend wird die Anzahl der Eier sowie die Anzahl der sich bewegenden Larven bestimmt und der Motiliäts-Index berechnet. Der Motilitäts-Index der Probe ergibt sich aus dem Mittelwert der 4 Einzelmessungen.

4.3 Reproduzierbarkeit und Präzision - Vergleich mit dem Infektivitätstest

Zur Überprüfung der Reproduzierbarkeit des Motilitätstests unter den oben beschriebenen Bedingungen wurden 4 analoge Proben einer *Trichuris suis*-Charge an 4 unterschiedlichen Tagen untersucht. Die Messergebnisse sind in den folgenden Tabellen aufgeführt. Als Vergleich sind die Ergebnisse von 4 Messreihen mit dem Infektivitätstest aufgeführt, die mit der selben *Trichuris suis*-Charge erhalten wurden.

Tab. 7: Bestimmung des Motilitäts-Index einer *Trichuris suis*-Charge (Ergebnisse von 4 unabhängigen Untersuchungen bestehend aus jeweils 4 Messungen)

| Motilitätstest | | | | | Infektivitätstest | | | | |
|---|---|---|---|---|---|---|---|---|---|
| Messreihe | Messung | Motilitäts-Index (%) | | | Messreihe | Versuchstier | Infektivitätsrate (%) | | |
| | | Einzelmessung | Mittelwert | Präzision | | | Einzelmessung | Mittelwert | Präzision |
| 1 | #1 | 78,4 | 79,1 ± 2,1 | 2,7% | 1 | #1 | 27,4 | 25.9 ± 2.5 | 9,6% |
| | #2 | 77,5 | | | | #2 | 24,7 | | |
| | #3 | 78,2 | | | | #3 | 23,5 | | |
| | #4 | 82,2 | | | | #4 | 29,5 | | |
| | | | | | | #5 | 24,5 | | |
| 2 | #1 | 73,0 | 79,6 ± 5,0 | 6,3% | 2 | #1 | 7,4 | 22.6 ± 13.2 | 58,4% |
| | #2 | 82,4 | | | | #2 | 29,9 | | |
| | #3 | 84,3 | | | | #3 | 26,7 | | |
| | #4 | 78,5 | | | | #4 | 10,7 | | |
| | | | | | | #5 | 38,6 | | |
| 3 | #1 | 87,5 | 82,4 ± 5,1 | 6,2% | 3 | #1 | 17,1 | 32,2 ± 11,8 | 36,6% |
| | #2 | 85,4 | | | | #2 | 33,1 | | |
| | #3 | 80,8 | | | | #3 | 49,9 | | |
| | #4 | 76,0 | | | | #4 | 31,8 | | |
| | | | | | | #5 | 29,1 | | |
| 4 | #1 | 81,1 | 79,8 ± 1,5 | 1,9% | 4 | #1 | 23,4 | 23,8 ± 2,9 | 12,1% |
| | #2 | 80,2 | | | | #2 | 23,7 | | |
| | #3 | 77,6 | | | | #3 | 27,9 | | |
| | #4 | 80,3 | | | | #4 | 24,1 | | |
| | | | | | | #5 | 19,8 | | |

Die Motilitäts-Indizes aus 4 unabhängigen Messreihen weichen um maximal 4,2% voneinander ab, dagegen liegt die maximale Abweichung der Infektivitätsrate aus 4 unabhängigen Messreihen bei 35,5%. Die Präzision der Einzelmessungen liegt beim Motilitätstest zwischen 1,9% und 6,3%, beim Infektivitätstest dagegen zwischen 9,6% und 58,4%. Der Motilitätstest ist dem Infektivitätstest also sowohl in der Reproduzierbarkeit als auch in der Präzision weit überlegen.

4.4 Korrelation zwischen Motilität und biologischer Aktivität (Richtigkeit) - Vergleich mit dem Infektivitätstest

Zur Überprüfung der Korrelation zwischen Motilität und biologischer Aktivität wurden Mischungen aus aktiven TSO und thermisch inaktivierten TSO hergestellt und mit dem Motilitätstest nach den in 4.2 beschriebenen Bedingungen untersucht. Die thermische Inaktivierung erfolgte durch Erhitzen der Eier bei 48°C über 72 Stunden. Um objektive Zählergebnisse zu erhalten, wurden die Proben vor der Messung verblindet.

Die relative biologische Aktivität wurde als Quotient aus dem gemessenen Motilitätsindex der Probe und dem zuvor bestimmten Motilitätsindex der aktiven TSO berechnet. Die Richtigkeit der Messung ergibt sich aus dem Vergleich relativen biologischen Aktivität und dem tatsächlichen Anteil der aktiven Eier in der Probe.

Eine analoge Messreihe mit TSO aus der gleichen Charge wurde mit dem Infektivitätstest durchgeführt. Die Versuchstiere wurden mit unterschiedlichen Mengen aktiver TSO mit bekannter Infektivitätsrate infiziert. Auf eine Zumischung von inaktivierten Eiern wurde verzichtet. Die Ergebnisse sind in den nächsten Tabellen dargestellt.

Tab. 8: Richtigkeit und Präzision des Motilitätstests

| Probe | 1 | 2 | 3 | 4 | 5 | 6 |
|---|---|---|---|---|---|---|
| Anteil aktive TSO in der Probe = Erwartungswert der rel. biol. Aktivität | 0% | 30,0% | 50% | 70% | 90,0% | 100% |
| Motilitätsindex, Messung #1 | 0% | 25,2% | 39,6% | 56,1% | 72,7% | 89,8% |
| Motilitätsindex, Messung #2 | 0% | 26,5% | 39,8% | 56,5% | 78,3% | 86,3% |
| Motilitätsindex, Messung #3 | 0% | 20,6% | 42,7% | 55,3% | 79,1% | 87,3% |
| Motilitätsindex, Messung #4 | 0% | 20,0% | 43,5% | 58,9% | 76,9% | 87,0% |
| Motilitätsindex, Mittelwert | 0% | 23,1% | 41,4% | 56,7% | 76,8% | 87,6% |
| Motilitätsindex, 100% aktive TSO | 80,2% | 80,2% | 80,2% | 80,2% | 80,2% | 80,2% |
| Rel. Biologische Aktivität | 0% | 28,8% | 51,6% | 70,7% | 95,7% | 109,2% |
| Richtigkeit (Abweichung vom Erwartungswert) | ± 0% | - 4,1% | + 3,3% | + 1,0% | + 6,4% | + 9,2% |
| Präzision (Streuung der Messwerte) | n.a. | ± 14,2% | ± 4,8% | ± 2,8% | ± 3,7% | ± 1,7% |

Tab. 9: Richtigkeit und Präzision des Infektivitätstests

| Probe | 1 | 2 | 3 | 4 |
|---|---|---|---|---|
| Anteil aktive TSO in der Probe = Erwartungswert der rel. biol. Aktivität | 0% | 25,0% | 50,0% | 100,0% |
| Infektivitätsrate, Versuchstier #1 | 0% | 4,1% | 12,4% | 52,2% |
| Infektivitätsrate, Versuchstier #2 | 0% | 6,2% | 17,2% | 6,3% |
| Infektivitätsrate, Versuchstier #3 | 0% | 0,4% | 20,4% | 31,1% |
| Infektivitätsrate, Versuchstier #4 | 0% | 9,5% | 22,6% | 36,0% |
| Infektivitätsrate, Versuchstier #5 | | 2,3% | 11,8% | 37,9% |
| Infektivitätsrate, Mittelwert | 0% | 4,5% | 16,9% | 32,7% |
| Infektivitätsrate, 100% aktive TSO | 26,1% | 26,1% | 26,1% | 26,1% |
| Rel. Biologische Aktivität | 0% | 17,0% | 64,6% | 125,2% |
| Richtigkeit (Abweichung vom Erwartungswert) | ± 0% | - 31,8 % | + 29,1% | + 25,2% |
| Präzision (Streuung der Messwerte) | n.a. | ± 79,2% | ± 28,4% | ± 51,1% |

Mit dem hier dargestellten Motilitätstest lässt sich die relative biologische Aktivität einer TSO-Probe über den gesamten Bereich zwischen 0% und 100% mit einer Abweichung von < 10% vom Erwartungswert richtig bestimmen. Die Präzision der Messung (Streuung der Messwerte) ist im Bereich zwischen 50% und 100% relativer biologischer Aktivität kleiner als 5%. Bei Bestimmungen niedrigerer relativer biologischer Aktivitäten liegt die Präzision unter 15%.

Die Ergebnisse des Infektivitätstests weichen im Bereich zwischen 0% und 100% relativer biologischer Aktivität um ca. 25-32% vom Erwartungswert ab. Die Streuung der Messwerte liegt zwischen 28% und 80%.

Das Beispiel zeigt eindeutig, dass der Motilitätsindex linear mit der relativen biologischen Aktivität korreliert. Bezüglich der Richtigkeit und Präzision ist der Motilitätstest dem Infektivitättstest weit überlegen.

Ein geeigneter Bereich für den Motilitätsindex als Maß für die biologische Aktivität von Helminth-Ei-Präparationen liegt bei 30% - 100%, bevorzugt 60 - 100%.

Beispiel 5:

Beispielhaft wurden Kaninchen verschiedene Mischungen aus embryonierten, nicht-embryonierten und inaktivierten, embryonierten Eiern oral verabreicht. Nach 8 Stunden wurde der Darminhalt der Versuchtiere mikroskopisch untersucht und die intakten Eier sowie die leeren Eihüllen wurden ausgezählt. Da die Eier in diesem Versuch nicht fluoreszenzmarkiert waren, wurde die Schlüpfrate aus dem Verhältnis der intakten embryonierten und nicht-embryonierten Eiern berechnet (nach der Formel oben mit [IE] = Anzahl embryonierter Eier und [IS] = Anzahl nicht-embryonierter Eier). Die nachfolgende Tabelle gibt die Ergebnisse der Untersuchung sowie die daraus berechnete Schlüpf-Rate wieder:

Tab. 10: Anzahl der intakten nicht-embryonierten und embryonierten *Trichuris suis* Eier, sowie der leeren Ei-Hüllen im Darminhalt von Kaninchen 8h nach oraler Verabreichung von Mischungen embryonierter, nicht-embryonierter und inaktivierter Eier

| Gruppe | Inokulierungsdosis | | | Eier/Eihüllen im Darminhalt | | | Schlüpfrate |
|---|---|---|---|---|---|---|---|
| | EE | IEE | NEE | EE | NEE | ES | |
| #1 | 36.000 | | 44.000 | 538 | 2050 | 2681 | 67.9% |
| #2 | | 36.000 | 44.000 | 7525 | 8166 | 65 | -12,6% |
| #3 | | | 80.000 | | 3860 | 15 | n.a. |
| (EE: intakte embryonierte Eier; IEE: intakte hitze-inaktivierte embryonierte Eier; NEE: intakte nicht-embryonierte Eier; ES: leere Ei-Hüllen; n.a.: nicht anwendbar) | | | | | | | |

Der Vergleich der Daten aus den Gruppen 1-3 zeigt, dass nur aktive embryonierte Eier in der Lage sind, im Kaninchen zu schlüpfen (erkennbar durch die deutliche Abnahme der Anzahl intakter embryonierter Eier und der Anwesenheit einer großen Anzahl leerer Ei-Hüllen). Dagegen bleiben inaktivierte embryonierte Eier im Intestinum unverändert (Gruppe 2). Dies belegt eindeutig, dass man mit dem Verfahren zwischen biologisch aktiven und inaktivierten Eiern differenzieren kann. Zudem bleiben auch nicht-embryonierte Eier bei der Darm-Passage intakt (Gruppe 3). Dies erlaubt eine Nutzung von nicht-embryonierten Eiern als interner Standard, der den Darm der Versuchstiere durchläuft ohne abgebaut zu werden. Dieser interne Standard ist notwendig, da die Wiederfindung der Eier im Darminhalt nur unvollständig ist und somit die absolute Anzahl der wiedergefundenen intakten Eier nicht aussagekräftig ist. Zur Berechnung der Schlüpfrate unter Berücksichtigung des internen Standards dient die oben angegebene Formel. Nach dieser Formel zeigen die hier untersuchten *Trichuris suis* Eier eine Schlüpfrate von 67.9%, während die inaktivierten *Trichuris suis* Eier eine Schlüpfrate von 0% (rechnerisch: -12.6%) zeigen.

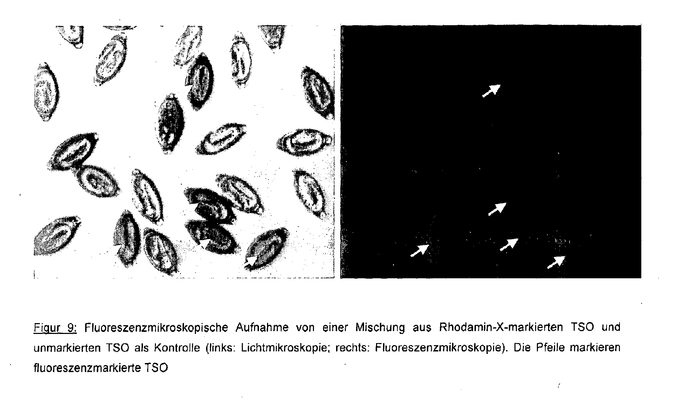

Um eine bessere Wiederfindung und eine klarere Differenzierung zwischen den Eiern der Probe und den Eiern des internen Standards zu erzielen, wurden *Trichuris suis* Eier mit einer Fluoreszenz-Sonde kovalent markiert. Beispielhaft wurde als Fluoreszenzsonde Rhodamin X - succinimidyl - ester eingesetzt, das nach Angaben des Herstellers mit primären Aminogruppen in Proteinen reagieren kann. Eine Suspension von 1000 Trichuris suis Eiern in 200 $\mu$l Phosphatpuffer, pH 7.4, wurde mit 20 $\mu$l einer 1 M Natriumbicarbonatlösung versetzt und anschließend mit 5 $\mu$l einer 0.5%-igen Lösung von Rhodamin X - succinimidyl - ester in DMSO umgesetzt. Die Lösung wurde unter Schwenken 1 h bei Raumtemperatur inkubiert. Anschließend wurden die Eier insgesamt 8 mal durch Zentrifugation (10 min bei 500 rpm) und Austausch des überstehenden Puffers gereinigt. Die Fluoreszenzmikroskopie zeigt eindeutig die Rotfärbung der Ei-Hülle (Fig. 9).

Die Fluoreszenzmarkierung lässt sich vorteilhaft zur mikroskopischen Auswertung der Schlüpfrate nutzen. Sie bietet darüber hinaus auch die Möglichkeit zur objektiven und schnellen Analyse mittels fluoreszenzaktivierter Durchflußzytometrie.

Insgesamt zeigt das Beispiel eindeutig, dass sich mit dem hier dargestellten Verfahren die die Phase des Schlüpfens quantitativ untersuchen und zur Bestimmung der biologischen Aktivität nutzen lässt. Ein geeigneter Bereich für die Schlüpfrate als Maß für die biologische Aktivität von Helminth-Ei-Präparationen liegt bei 25%-100%.

**Patentansprüche**

1.  Verfahren zur Bestimmung der biologischen Aktivität von Helminth-Eiern, bei der wenigstens eine der nachfolgenden Bestimmungen durchgeführt wird:

a) Bestimmung des Stadiums der Embryonalentwicklung von Helminth-Eiern mit Hilfe der quantitativen PCR-Analyse unter Verwendung geeigneter Marker-Sequenzen zur Ermittlung der Kopienzahl der genomischen DNA,
b) Bestimmung der metabolischen Aktivität von embryonierten Helminth-Eiern mit biochemischen und/oder molekularbiologischen Methoden,
c) Bestimmung der Induzierbarkeit der Genexpression in embryonierten Helminth-Eiern,
d) mikroskopische Bestimmung der Motilität von im Ei befindlichen Helminth-Larven über lange Beobachtungszeiträume nach Vorinkubation bei erhöhten Temperaturen, und/oder
e) Bestimmung der Schlüpfrate von Helminth-Larven im Versuchstier, wobei die aus dem Darminhalt zurückgewonnenen intakten embryonierten Eier im Vergleich zu einem internen Standard quantifiziert werden.

2. Verfahren nach Anspruch 1a), bei dem mittels quantitativer PCR-Analyse unter Verwendung geeigneter, für *Trichuris suis* spezifischer Sequenzen die Kopienzahl der genomischen DNA bestimmt wird.

3. Verfahren gemäß Anspruch 1b), **dadurch gekennzeichnet, dass** zur Bestimmung der metabolischen Aktivität von embryonierten Helminth-Eiern der ATP-Gehalt gemessen wird.

4. Verfahren gemäß Anspruch 3, **dadurch gekennzeichnet, dass** die Helminth-Eier vor der Luminiszenzmessung unter folgenden Bedingungen vorinkubiert werden:

aa) zwischen 36°C und 42°C
bb) zwischen 2 und 30 Stunden
cc) in einem Suspensionsmedium mit einem pH-Wert zwischen 0.1 und 3.

5. Verfahren gemäß Anspruch 1b), **dadurch gekennzeichnet, dass** die Helminth-Eier zunächst mit einem Vorbehandlungsmittel ausgewählt aus hypochloriger Säure, Chitinase und/oder Protease behandelt werden und dann mit Tetrazoliumsalzen angefärbt werden.

6. Verfahren gemäß Anspruch 1c), **dadurch gekennzeichnet, dass** die Induzierbarkeit eines Hitzeschockproteins bestimmt wird.

7. Verfahren gemäß Anspruch 1c), **dadurch gekennzeichnet, dass** der Nachweis der Expression durch Hybridisierung mit einer fluoreszenzmarkierten Nucleotid-Sonde erfolgt.

8. Verfahren gemäß Anspruch 1c), **dadurch gekennzeichnet, dass** die Hybridisierung mit Hilfe der Durchflußzytometrie nachgewiesen wird.

9. Verfahren gemäß Anspruch 1d), **dadurch gekennzeichnet, dass** die Motilität der im Ei befindlichen Helminth-Larven mikroskopisch über Zeiträume von 2 min bis 8 Stunden mit Hilfe von Zeitrafferaufnahmen bestimmt wird.

10. Verfahren gemäß Anspruch 1e), **dadurch gekennzeichnet, dass** die zu überprüfenden Helminth-Eier mit Fluoreszenzsonden markiert werden und die internen Standards mit andersfarbigen Fluoreszenzsonden markiert werden.

11. Verfahren gemäß Anspruch 1e), **dadurch gekennzeichnet, dass** der Darminhalt von Kaninchen und/oder Schweinen als Prüfsystem verwendet wird.

12. Verfahren gemäß Anspruch 1e), **dadurch gekennzeichnet, dass** als interner Standard für die Ermittlung der Hatching-Rate nicht-embryonierte oder inaktivierte Helminth-Eier verwendet werden.

13. Verfahren nach einem der vorhergehenden Ansprüche, **dadurch gekennzeichnet, dass** wenigstens drei Bestimmungen ausgewählt unter den Bestimmungen 1a), 1b), 1c), 1d) und/oder 1e) durchgeführt werden.

14. Verfahren nach einem der vorhergehenden Ansprüche, **dadurch gekennzeichnet, dass** die Helminth-Eier vor der Durchführung der Bestimmung über einen Zeitraum von wenigstens 30 Minuten bis 24 Stunden unter genau standardisierten Bedingungen vorinkubiert wurden, wobei die Vorinkubation gegebenenfalls auch eine Änderung eines wesentlichen Parameters, insbesondere der Temperatur, beinhaltet.

15. Helminth-Eier, **dadurch gekennzeichnet, dass** sie zu wenigstens 50 % biologisch aktiv sind, bestimmt nach einem Verfahren nach einem der Ansprüche 1-14, und die Embryonalentwicklung durchlaufen können.

16. Verwendung von Helminth-Eiern gemäß Anspruch 15 zur Herstellung eines pharmazeutischen Präparats zur Behandlung einer Autoimmunerkrankung.

Forward Primer

TAGCAGCGACGGCAGGTGCCCGTCATCGCTGGCAGGCAGCCGGAGCTGCGGAGAGCGGCTAACTCAGC

GCAGTACGGAAGCTGCCCGAGTTGGCTACGTCGTCGCTACATCGTCGTCAGCCGTACAGCGCGACTGAG

TaqMan-Probe

Reverse Primer

Figur 1: Ausschnitt aus der ITS2-Sequenz von *T. suis* mit Primern und TaqMan-Probe für die quantitative PCR

$$\text{Luciferin} + \text{ATP} + O_2 \xrightarrow[\text{Mg}^{2+}]{\text{Luciferase}} \text{Oxyluciferin} + \text{AMP} + \text{PPi} + h\nu$$

Figur 2: Luciferase-Reaktion zum quantitativen Nachweis von Adenosintriphosphat

Erläuterungen zu Fig. 3:

- Aktiv ohne Preincubation:      Nicht inaktivierte Eier ohne Vorbehandlung bei 37°C über 19 Stunden
  (Eier im Ruhezustand bei 2-8°C)
- Inaktiv ohne Preincubation:    Kryoinaktivierte Eier ohne Vorbehandlung bei 37°C über 19 Stunden
- Aktiv ohne Preincubation:      Nicht inaktivierte Eier mit Vorbehandlung bei 37°C über 19 Stunden
- Inaktiv ohne Preincubation:    Kryoinaktivierte Eier mit Vorbehandlung bei 37°C über 19 Stunden

Figur 3: Luciferase-Reaktion zum quantitativen Nachweis von Adenosintriphosphat

Figur 4: Standardkurve von Adenosintriphosphat

```
T.suis_aa              ------------IGRRYDDAAVQSDMKHWPFKVVSDGGKPKIQVEYKGETKMFTPEEVS 47
C.elegans_aa      RNPENTVFDAKRLIGRRFDEETVQSDIKHWPFTVKGKQGKPVVBVEVKGEKREFNAEHIS 120
                         ****;*; ;****;*****.*  ,,  *** ;;** ***., *.,**;*

T.suis_aa         AMVLVKMKETAEAYLG----------------------------------------- 63
C.elegans_aa      AMVLQKMKETAEAVLGHSVRDAVITVPAYFNDSQRQATKDAATIAGLNAIRIINEPTAAA 180
                  **** ********* **
```

Figur 5: Vergleich der Proteinsequenz des putativen Hitzeschock-Proteins aus *T. suis* (Ausschnitt) mit hsp70 aus *C.elegans* (gleiche Basen:"*"; Basen mit ähnlichen physikochemischen Eigensschaften "." oder ":"):

```
CTCCGTCACTGACCACCTTGAAAGGCCAATGCTTCATGTCAGACTG
```

Figur 6: FISH-Sonde zum Nachweis der Expression von Hitzeschock-Protein mRNA in aktiven embryonierten Eiern von *T.suis* (T: Allyl-Amino-Thymin-Basen, an die Fluorophore gekoppelt sind)

Figur 7: Motilitätsindex von aktiven und thermisch inaktivierten *Trichuris suis* Eiern nach Inkubation bei 39°C

Figur 8: Motilitätsindex einer *Trichuris suis*-Präparation nach verschieden langen Beobachtungszeiträumen bei 39.5°C

Figur 9: Fluoreszenzmikroskopische Aufnahme von einer Mischung aus Rhodamin-X-markierten TSO und unmarkierten TSO als Kontrolle (links: Lichtmikroskopie; rechts: Fluoreszenzmikroskopie). Die Pfeile markieren fluoreszenzmarkierte TSO

EP 2 123 774 A1

**EUROPÄISCHER RECHERCHENBERICHT**

Europäisches Patentamt
European Patent Office
Office européen des brevets

Nummer der Anmeldung

EP 08 00 9344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | PECSON BRIAN M ET AL: "A real-time PCR method for quantifying viable ascaris eggs using the first internally transcribed spacer region of ribosomal DNA." APPLIED AND ENVIRONMENTAL MICROBIOLOGY DEC 2006, Bd. 72, Nr. 12, Dezember 2006 (2006-12), Seiten 7864-7872, XP002501996 ISSN: 0099-2240 * Zusammenfassung * ----- | 1,2,14 | INV. C12Q1/68 |
| X | WO 2007/076868 A (PARASITE TECHNOLOGIES AS [DK]; KAPEL CHRISTIAN MOLLIN OUTZEN [DK]; ROE) 12. Juli 2007 (2007-07-12) * Anspruch 13 * ----- | 15,16 | |
| D,A | WO 2007/134761 A (OVAMED GMBH [DE]; ABROMEIT NORBERT [DE]) 29. November 2007 (2007-11-29) * Ansprüche * ----- | | |
| D,A | KRINGEL ET AL: "Trichuris suis population dynamics following a primary experimental infection" VETERINARY PARASITOLOGY, ELSEVIER SCIENCE, AMSTERDAM, NL, Bd. 139, Nr. 1-3, 30. Juni 2006 (2006-06-30), Seiten 132-139, XP005460788 ISSN: 0304-4017 * Absatz [02.2] * ----- -/-- | | RECHERCHIERTE SACHGEBIETE (IPC) C12Q |

Der vorliegende Recherchenbericht wurde für alle Patentansprüche erstellt

| Recherchenort | Abschlußdatum der Recherche | Prüfer |
|---|---|---|
| Den Haag | 31. Oktober 2008 | Reuter, Uwe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 08 00 9344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | SUMMERS ET AL: "Trichuris suis therapy for active ulcerative colitis: A randomized controlled trial" GASTROENTEROLOGY, ELSEVIER, PHILADELPHIA, PA, Bd. 128, Nr. 4, 1. April 2005 (2005-04-01), Seiten 825-832, XP005110857 ISSN: 0016-5085 * Seite 826, linke Spalte, letzter Absatz * ----- | | |
| A | JOHNSON P W ET AL: "An in-vitro test for assessing the viability of Ascaris suum eggs exposed to various sewage treatment processes." INTERNATIONAL JOURNAL FOR PARASITOLOGY APR 1998, Bd. 28, Nr. 4, April 1998 (1998-04), Seiten 627-633, XP008097926 ISSN: 0020-7519 * Absatz [0004] * ----- | | |
| A | BOES J ET AL: "Embryonation and infectivity of Ascaris suum eggs isolated from worms expelled by pigs treated with albendazole, pyrantel pamoate, ivermectin or piperazine dihydrochloride" VETERINARY PARASITOLOGY, ELSEVIER SCIENCE, AMSTERDAM, NL, Bd. 75, Nr. 2-3, 28. Februar 1998 (1998-02-28), Seiten 181-190, XP002449735 ISSN: 0304-4017 * Absatz [02.5] * ----- | | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 31. Oktober 2008 | Reuter, Uwe |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
..............................................................................................
& : Mitglied der gleichen Patentfamilie, übereinstimmendes
   Dokument

EPO FORM 1503 03.82 (P04C03)

Europäisches
Patentamt
European
Patent Office
Office européen
des brevets

**Nummer der Anmeldung**

EP 08 00 9344

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

siehe Anhang

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

| Europäisches Patentamt  European Patent Office  Office européen des brevets | **MANGELNDE EINHEITLICHKEIT DER ERFINDUNG ERGÄNZUNGSBLATT B** | **Nummer der Anmeldung**  EP 08 00 9344 |

Nach Auffassung der Recherchenabteilung entspricht die vorliegendeeuropäische Patentanmeldung nicht den Anforderungen an die Einheitlichkeit der Erfindungund enthält mehrere Erfindungen oder Gruppen von Erfindungen, nämlich:

1. Ansprüche: 1,14-16 (alle teilweise), 2 (vollständig)

   Verfahren zur Bestimmung der biologischen Aktivität von Helminth-Eiern durch Bestimmung des Stadiums der Embryonalentwicklung von Helminth-Eiern mit Hilfe der quantitativen PCR-Analyse, Helminth- Eier und deren Verwendung

   ---

2. Ansprüche: 1,13-16 (alle teilweise), 3-5 (vollständig)

   Verfahren zur Bestimmung der biologischen Aktivität von Helminth-Eiern durch Bestimmung der metabolischen Aktivität von embryonierten Helminth-Eiern, Helminth- Eier und deren Verwendung

   ---

3. Ansprüche: 1,13-16 (alle teilweise), 6-8 (vollständig)

   Verfahren zur Bestimmung der biologischen Aktivität von Helminth-Eiern durch Bestimmung der Induzierbarkeit der Genexpression in embryonierten Helminth-Eiern, Helminth- Eier und deren Verwendung

   ---

4. Ansprüche: 1,13-16 (alle teilweise), 9 (vollständig)

   Verfahren zur Bestimmung der biologischen Aktivität von Helminth-Eiern durch mikroskopische Bestimmung der Motilität von im Ei befindlichen Helminth-Larven, Helminth- Eier und deren Verwendung

   ---

5. Ansprüche: 1,13-16 (alle teilweise), 10-12 (vollständig)

   Verfahren zur Bestimmung der biologischen Aktivität von Helminth-Eiern durch Bestimmung der Schlüpfrate von Helminth-Larven im Versuchstier, Helminth- Eier und deren Verwendung

   ---

**ANHANG ZUM EUROPÄISCHEN RECHERCHENBERICHT**
**ÜBER DIE EUROPÄISCHE PATENTANMELDUNG NR.**

EP 08 00 9344

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-10-2008

| Im Recherchenbericht angeführtes Patentdokument | Datum der Veröffentlichung | Mitglied(er) der Patentfamilie | Datum der Veröffentlichung |
|---|---|---|---|
| WO 2007076868 A | 12-07-2007 | AU 2006332241 A1<br>CA 2631566 A1<br>CN 101346150 A<br>EP 1968618 A2 | 12-07-2007<br>12-07-2007<br>14-01-2009<br>17-09-2008 |
| WO 2007134761 A | 29-11-2007 | DE 102006023906 A1<br>EP 2027464 A1<br>US 2007269795 A1 | 22-11-2007<br>25-02-2009<br>22-11-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 199933479 A
- WO 2007076868 A
- WO 2007134761 A


**In der Beschreibung aufgeführte Nicht-Patentliteratur**

- **D M McKay.** *Parasitology,* 2006, vol. 132, 1-12
- **Summers et al.** *Am J Gastroenterol,* 2003, vol. 98, 2034-2041
- **Beer.** *Br. Med. J.,* 1971, vol. 3, 41
- **Summers et al.** *Am. J. Gastroenterol.,* 2003, vol. 98, 2034-2041
- **Beer.** *Parasitol,* 1973, vol. 67, 253-262
- **Kringel et al.** *Vet. Parasitol.,* 2006, vol. 139, 132-139
- **Cutillas et al.** *Parasitol Res,* 2001, vol. 100, 383-389
- **Beer.** *Parasitol,* 1973, vol. 67, 263-278
- *Parasitology,* 1973, vol. 67, 253-262